# FASCICULE DE BREVET EUROPEEN

(11) **EP 0 658 546 B1**
(45) Date de publication et mention de la délivrance du brevet: **23.05.2001**
(21) Numéro de dépôt: 94402890.1
(22) Date de dépôt: 15.12.1994
(51) Int. Cl.: C07D 231/14, C07D 401/12, A61K 31/415, C07D 403/12, C07D 453/02

(54) **Dérivés de 3-pyrazolecarboxamide avec une affinité pour le récepteur des cannabinoides**
3-Pyrazelcarboxamidderivate mit Cannabinoid-Rezeptor-Affinität
Novel 3-pyrazolecarboxamide derivatives with cannabinoid receptor affinity

(30) Priorité: 17.12.1993 FR 9315221
(43) Date de publication de la demande: 21.06.1995
(73) Titulaire: SANOFI-SYNTHELABO, 75013 Paris (FR)
(72) Inventeur: Barth, Francis, F-34080 Montpellier (FR); Casellas, Pierre, F-34000 Montpellier (FR); Congy, Christian, F-34980 Saint Gely du Fesc (FR); Martinez, Serge, F-34000 Montpellier (FR); Rinaldi, Murielle, F-34680 Saint Georges d'Orgues (FR)
(74) Mandataire: Gillard, Marie-Louise

(56) Documents cités:
- EP-A- 0 445 781
- EP-A- 0 477 049
- EP-A- 0 576 357
- US-A- 3 449 350
- CHEMICAL ABSTRACTS, vol. 86, no. 17, 25 Avril 1977, Columbus, Ohio, US; abstract no. 120940d, S.A. KHATTAB ET AL. 'Structure of the diazonium coupling products of gamma-phenyl-delta-butenolide.' page 512 ;colonne 2 ; & J. CHEM. ENG. DATA, vol.22, no.1, 1977 pages 104 - 110
- CHEMICAL ABSTRACTS, vol. 59, no. 3, 5 Août 1963, Columbus, Ohio, US; A. MUSTAFA ET AL. 'Behavior of the hetero ring in gamma-phenyl-delta- butenolide derivatives towards hydrazines. Acid rearrangement of 4-phenyl-hydrazono-2-phen yloxazolin-5-one.' colonne 2815H ; & CAN. J. CHEM., vol.41, no.7, 1963 pages 1813 - 1818

## Description

La présente invention concerne de nouveaux dérivés du 3-pyrazolecarboxamide et leurs sels, un procédé pour leur préparation et des compositions pharmaceutiques les contenant.

De nombreux dérivés du pyrazole ont été décrits dans la littérature ; plus particulièrement EP-A-268554 et DE-A-3910248 revendiquent des pyrazoles possédant des propriétés - herbicides, EP-A-430186 et JP-A-03031840 revendiquent des composés utiles pour la photographie et EP-A-418845 revendique des pyrazoles pourvus d'activité antiinflammatoire, analgésique et antithrombotique.

La demande EP-A-576 357 décrit entre autres des composés présentant une affinité pour le récepteur aux cannabinoïdes, de formule : 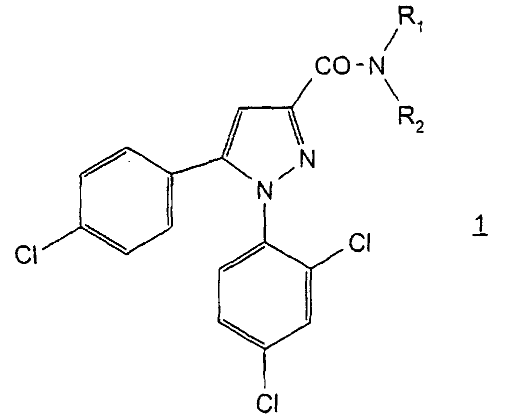 dans laquelle, notamment :
- R₁ représente l'hydrogène ou un (C₁-C₆)alkyle ;
- R₂ représente un radical hétérocyclique saturé de 5 à 8 chaînons non substitué, en particulier un radical pyrrolidin-1-yle, pipéridin-1-yle, perhydroazépin-1-yle ou morpholin-4-yle.

On a maintenant trouvé que les dérivés de 3-pyrazolecarboxamide objet de l'invention possèdent une très bonne affinité pour le récepteur des cannabinoïdes et sont utiles dans les domaines thérapeutiques où le cannabis est connu pour intervenir.

Le Δ⁹-THC (Δ⁹-tétrahydrocannabinol) est le principal constituant actif extrait de *Cannabis sativa* (Tuner, 1985 ; In Marijuana 84, Ed. Harvey, DY, IRL Press, Oxford).

Les effets des cannabinoïdes sont dûs à une intéraction avec des récepteurs spécifiques de haute affinité présents au niveau central (Devane et al., Molecular Pharmacology, 1988, 34, 605-613) et périphérique (Nye et al., J. Pharmacol. Exp. Ther., 1985, 234, 784-791 ; Kaminski et al., Molecular Pharmacology, 1992, 42, 736-742 ; Munro et al., Nature, 1993, 365, 61-65 ; Bouaboula et al., Eur. J. Biochem., 1993, 214, 173-180).

Les indications thérapeutiques des cannabinoïdes concernent des domaines variés tel que le système immunitaire, le système nerveux central, le système cardiovasculaire ou endocrinien (Hollister, Pharmacological Reviews, 1986, 38, 1-20, Renv and Sinha, Progress in Drug Research, 1991, 36, 71-114 et Consroe et Sandyk, in Marijuana/Cannabinoids, Neurobiology and Neurophysiology, p. 459 Eds L. Murphy and A. Barthe, CRC Press, 1992).

La caractérisation de ce récepteur a été rendue possible par la mise au point de ligands synthétiques spécifiques tels que les agonistes WIN 55212-2 (J. Pharmacol. Exp. Ther., 1993, 264, 1352-1363) ou 55,940 (J. Pharmacol. Exp. Ther., 1988, 247, 1046-1051).

Selon un de ses aspects, la présente invention concerne les composés de formule : 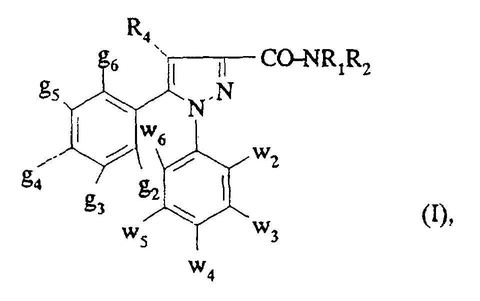 dans laquelle :
- g₂, g₃, g₄, g₅, g₆ et w₂, w₃, w₄, w₅, w₆ sont identiques ou différents, et représentent indépendamment l'hydrogène, un atome de chlore, de brome ou d'iode, un (C₁-C₃)alkyle, un (C₁-C₃)alcoxy, un trifluorométhyle, un groupe nitro et g₄ peut également représenter un groupe phényle ;
- R₁ représente un (C₁-C₆)alkyle ou un hydrogène ;
- R₂ représente -⁺NR₃R₅R₆ ou -NR₅R₆ ;
- R₃ représente un (C₁-C₆)alkyle ou R₃ forme un pont avec un des atomes du radical hétérocyclique formé par NR₅R₆ ;
- R₄ représente l'hydrogène ou un (C₁-C₅)alkyle ;
- R₅ et R₆ ensemble avec l'atome d'azote auquel ils sont liés constituent un radical hétérocyclique, saturé ou insaturé, de 5 à 10 chaînons, non substitué ou substitué une ou plusieurs fois par un alkyle en C₁-C₆, un benzyle, un phényle, un hydroxyle, un alcoxy en C₁-C₆, un halogène ;
à la condition que lorsque R₂ représente NR₅R₆, R₅ et R₆ ensemble avec l'atome d'azote auquel ils sont liés constituent un radical hétérocyclique autre qu'un radical saturé de 5 à 8 chaînons non substitué ou substitué par un (C₁-C₃)alkyle, un hydroxyle ou un benzyle ;
leurs sels ou leurs solvates.

Par (C₁-C₃)alkyle, (C₁-C₅)alkyle, (C₁-C₆)alkyle, on entend des alkyles droits ou ramifiés, respectivement en C₁-C₃, C₁-C₅ ou C₁-C₆. Les groupes alkyles préférés sont les groupes méthyle, éthyle, propyle et isopropyle.

Par radical hétérocyclique saturé ou insaturé de 5 ou 10 chaînons, on entend un radical hétérocyclique non aromatique mono, di ou tricyclique, condensé ou ponté, pouvant contenir un deuxième hétéroatome tel que l'azote, l'oxygène ou le soufre. Ces radicaux comprennent en particulier les radicaux suivants : 1-pyrrolidinyle, 1-pipéridinyle, 1-hexahydroazépinyle, 4-morpholinyle, 4-thiomorpholinyle, 2-azabicyclo[2.2.2]oct-5-èn-2-yle, 2-méthyl-2-azoniabicyclo[2.2.2]oct-5-èn-2-yle, 2-azaadamant-2-yle, 1,2,3,6-tétrahydropyridin-1-yle, 2-azabicyclo[2.2.1]heptan-2-yle, 2-azabicyclo[2.2.2]octan-2-yle, 1-azoniabicyclo[2.2.2]octan-1-yle.

Les sels du composé de formule (I) comprennent les sels d'addition d'acides pharmaceutiquement acceptables tels que le chlorhydrate, le bromhydrate, le sulfate, I'hydrogénosulfate, le dihydrogénophosphate, le méthanesulfonate, le méthylsulfate, le maléate, l'oxalate, le fumarate, le 2-naphtalènesulfonate, le glyconate, le gluconate, le citrate, l'iséthionate, le paratoluènesulfonate ou le mésitylènesulfonate.

Dans la formule (I) ci-dessus, de préférence au moins un des substituants w₂, w₃, w₄, w₅, w₆, g₂, g₃, g₄, g₅ et g₆ est autre que l'hydrogène.

Les composés (I) dans lesquels au moins un des substituants w₂, w₃, w₄, w₅, w₆, g₂, g₃, g₄, g₅ et g₆ est choisi parmi un chlore et un méthyle, sont préférés.

Particulièrement préférés sont les composés dans lesquels w₂, w₄ et g₄ sont des atomes de chlore.

Les composés (I) dans lesquels R₄ est H, méthyle ou éthyle sont préférés.

Les composés de formule (I) préférés sont ceux dans lesquels w₂, w₄ et g₄ représentent chacun un chlore, R₁ représente l'hydrogène, R₄ représente un méthyle ou un éthyle, et R₂ a l'une des valeurs indiquées ci-dessus pour (I).

D'autres composés (I) préférés selon l'invention sont ceux dans lesquels g₄ est un chlore ou un méthyle, w₃ et w₄ ou w₂ et w₅ sont choisis parmi chlore et méthyle, R₁ représente un méthyle, un éthyle ou un propyle, R₄ est l'hydrogène et R₂ a l'une des valeurs indiquées ci-dessus pour (I).

Les composés (I) dans lesquels R₂ représente un radical hétérocyclique monoinsaturé à 5, 6 ou 7 chaînons sont préférés.

Plus particulièrement, les composés (I) préférés sont ceux dans lesquels R₂ représente un groupe 1,2,3,6-tétrahydropyridin-1-yle.

Selon un autre de ses aspects, la présente invention concerne un procédé pour la préparation d'un composé (I), de ses sels et de leurs solvates, caractérisé en ce qu'on traite un dérivé fonctionnel de l'acide pyrazole-3-carboxylique de formule : 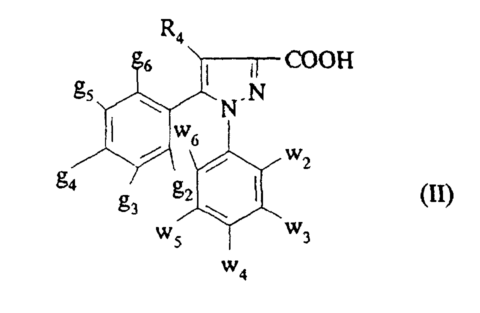 dans laquelle w₂, w₃, w₄, w₅, w₆, g₂, g₃, g₄, g₅, g₆ et R₄ sont tels que définis pour (I) avec une amine de formule HNR₁R₂ dans laquelle R₁ et R₂ sont tels que définis pour (I), et éventuellement on transforme le composé ainsi obtenu en l'un de ses sels ou de leurs solvates.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide ou avec l'hexafluorophosphate de benzotriazol-N-oxotris(diméthylamino)phosphonium (BOP).

Ainsi dans le procédé selon l'invention, on peut faire réagir le chlorure de l'acide pyrazole-3-carboxylique, obtenu par réaction du chlorure de thionyle sur l'acide de formule (II), avec une amine HNR₁R₂, dans un solvant tel que le dichlorométhane, sous une atmosphère inerte, à une température comprise entre 0°C et la température ambiante, en présence d'une base telle que la triéthylamine.

Une variante au mode opératoire consisté à préparer l'anhydride mixte de l'acide de formule (II) par réaction du chloroformiate d'éthyle avec l'acide de formule (II), en présence d'une base telle que la triéthylamine, et à le faire réagir avec une amine HNR₁R₂, dans un solvant tel que le dichlorométhane, sous une atmosphère inerte, à la température ambiante, en présence d'une base telle que la triéthylamine.

Le composé de formule (I) ainsi obtenu est isolé, sous forme de base libre ou de sel ou de solvate, selon les techniques conventionnelles.

Lorsque le composé de formule (I) est isolé sous forme d'un de ses sels, par exemple le chlorhydrate ou l'oxalate, la base libre peut être préparée par neutralisation dudit sel avec une base minérale ou organique, telle que l'hydroxyde de sodium ou d'ammonium, la triéthylamine ou un carbonate ou bicarbonate alcalin tel que le carbonate ou le bicarbonate de sodium ou de potassium, et transformée dans un autre sel comme le méthanesulfonate, le fumarate ou le 2-naphtalènesulfonate.

Lorsque le composé (I) est obtenu sous forme de base libre, la salification est effectuée par traitement avec l'acide choisi dans un solvant organique. Par traitement de la base libre, dissoute par exemple dans un éther tel que l'éther diéthylique ou dans l'acétone, avec une solution de l'acide dans le même solvant, on obtient le sel correspondant qui est isolé selon les techniques classiques.

Les composés de formule (I) dans lesquels R₂ représente -⁺NR₃R₅R₆ et R₃ représente un (C₁-C₆)alkyle s'obtiennent préférentiellement par réaction d'un halogénure d'alkyle (R₃Hal) dans lequel l'alkyle est en C₁-C₆ et l'halogène est choisi parmi le brome ou l'iode avec un composé de formule (I) dans lequel R₂ représente -NR₅R₆.

Les composés de formule (I) dans lesquels R₂ représente -⁺NR₃R₅R₆, et R₃ forme un pont avec un des atomes du radical hétérocyclique formé par NR₅R₆ s'obtiennent par action d'un composé de formule :

NH₂-N⁺R₃R₅R₆

sur un dérivé fonctionnel de l'acide pyrazole-3-carboxylique de formule (II).

L'acide de formule (II) utilisé comme composé de départ pour le procédé de la présente invention peut être préparé selon des méthodes classiques.

Selon un mode opératoire préférentiel, les dérivés de l'acide pyrazole-3-carboxylique de formule (II) peuvent être synthétisés à partir des esters correspondants.

Lorsque R₄ est H ou un groupe méthyle, lesdits esters sont synthétisés en appliquant la méthode décrite dans Berichte, 1887, 20, 2185 ; ils permettent de préparer les esters dans lesquels R₄ est un alkyle en C₂-C₅.

Le schéma réactionnel de préparation des composés (II) via leur ester méthylique ou éthylique (Alk = CH₃ ou C₂H₅) est représenté par le SCHEMA 1 ci-dessous.

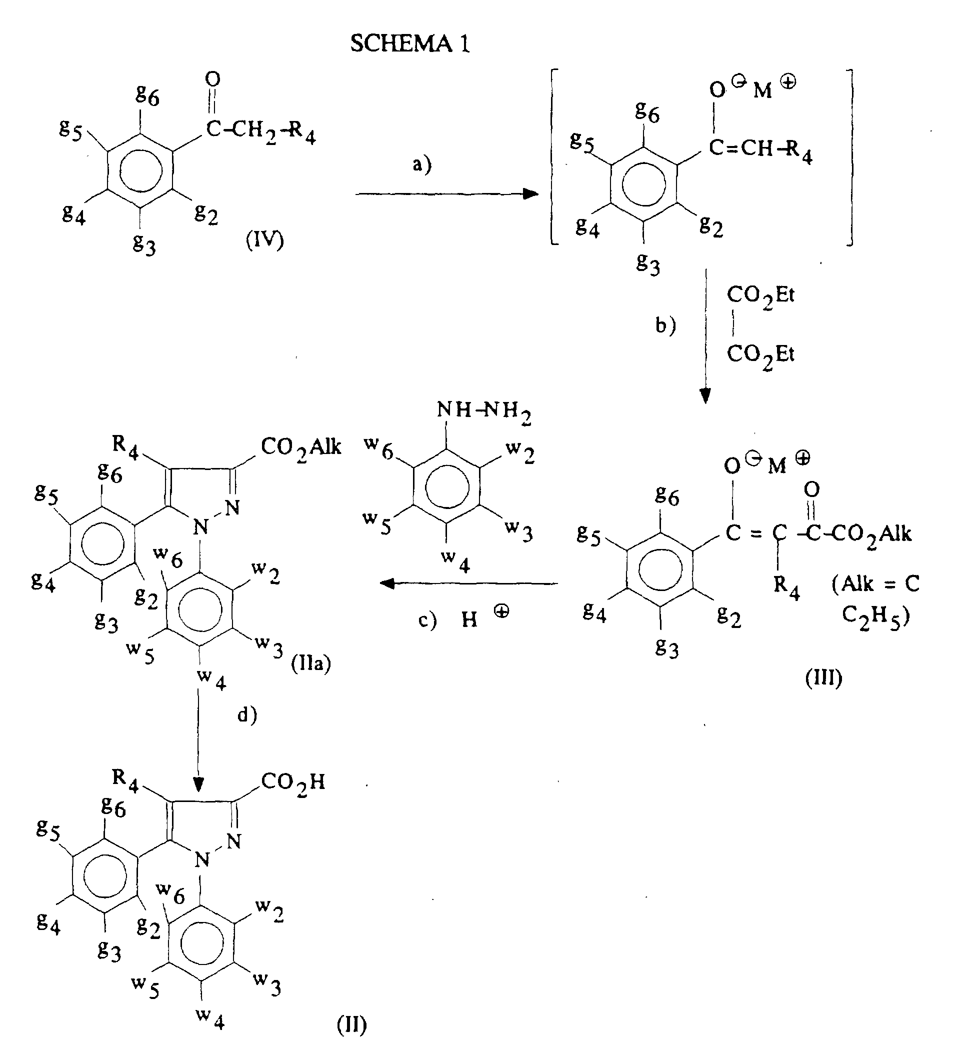

La première étape a) consiste en la préparation d'un sel de métal alcalin d'un dérivé de l'acétophénone de formule (IV) dans laquelle R₄ et g₂, g₃, g₄, g₅ et g₆ sont tels que définis ci-dessus pour (I) sur lequel est ensuite additionnée, une quantité équimolaire d'oxalate de diéthyle (étape b) pour obtenir le cétoester de formule (III).

Dans le cas où R₄ = H, le métal alcalin sera préférentiellement le sodium, et le sel du cétoester (III) (Alk = CH₃) sera obtenu selon la procédure décrite dans Bull. Soc. Chim. Fr., 1947, *14*, 1098 en utilisant le méthylate de sodium dans le méthanol pour effectuer l'étape a).

Dans le cas où R₄ = CH₃, le métal alcalin sera préférentiellement le lithium, et le sel du cétoester (III) (Alk = C₂H₅) sera obtenu selon J. Heterocyclic Chem. 1989, *26*, 1389 en utilisant le sel de lithium de l'hexaméthyldisilazane dans l'éther éthylique pour effectuer l'étape a).

Les sels de métal alcalin (III) ainsi préparés et un excès de dérivé de l'hydrazine sont alors chauffés au reflux de l'acide acétique (étape c). Par précipitation dans l'eau glacée on obtient ainsi les pyrazole-3-esters (IIa).

Ces esters (IIa) sont ensuite transformés en leurs acides (II) par action d'un agent alcalin comme par exemple l'hydroxyde de potassium puis acidification (étape d).

Préférentiellement lorsque R₄ représente un alkyle en C₂-C₅, on utilise le procédé décrit dans le schéma 2 ci-dessous pour la préparation des acides pyrazole-3-carboxylique de formule (II) :

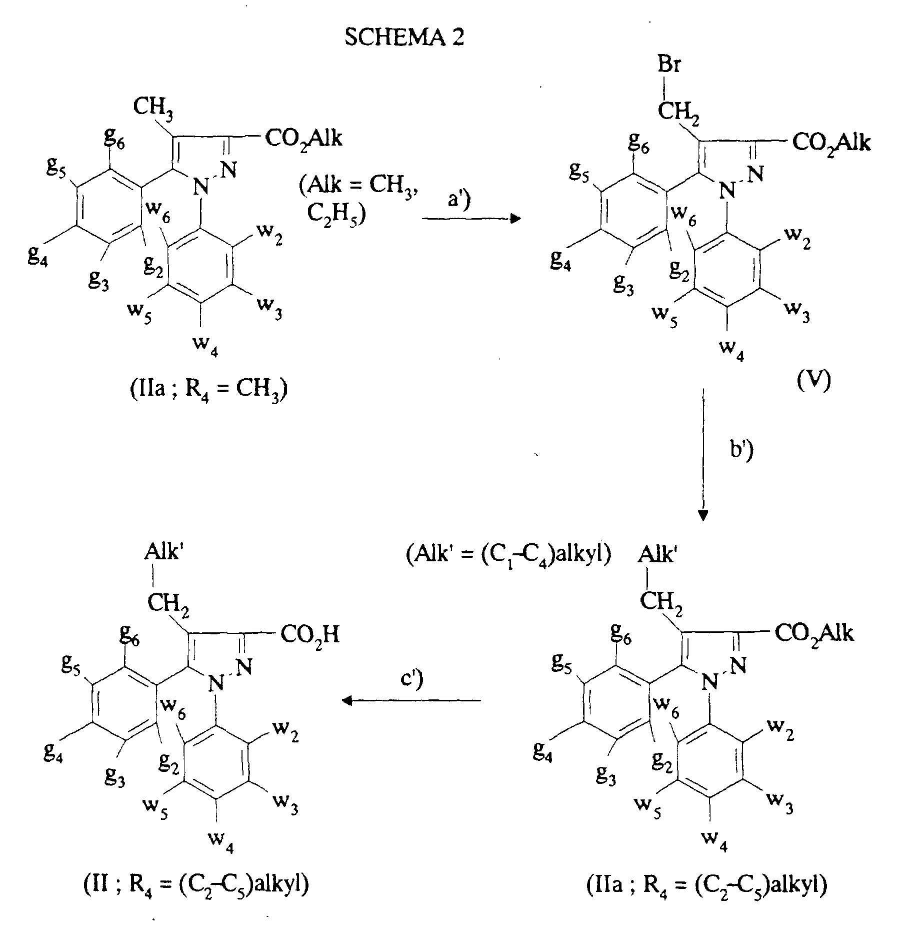

La première étape a') consiste en la préparation d'un 4-bromométhylpyrazole de formule (V) par action du N-bromosuccinimide sur un ester (IIa) dans lequel R₄ représente un CH₃. La réaction s'effectue dans un solvant comme le tétrachlorure de carbone en présence de péroxyde de dibenzoyle.

L'étape b') consiste en la préparation d'un ester (IIa) dans lequel R₄ représente un (C₂-C₅)alkyle par action d'un organocuprate (Alk')₂CuLi dans lequel Alk' est un alkyle en C₁-C₄ dans un solvant comme l'éther diéthylique à une température inférieure ou égale à -20°C.

Cet ester (IIa) est ensuite transformé en son acide (Il) par action d'un agent alcalin comme par exemple l'hydroxyde de potassium puis acidification (étape c').

Les amines HNR₁R₂ sont connues ou préparées par des méthodes connues.

Plus particulièrement on peut obtenir les amines HNR₁NR₅R₆ dans lesquelles R₁ = H selon le procédé décrit dans Chem. Ber., 1986, 119, 1413-1423, qui consiste en la réduction d'un dérivé nitroso de formule :

ON-NR₅R₆ (VI)

dans laquelle R₅ et R₆ sont tels que définis ci-dessus pour (I), par un hydrure tel que l'hydrure d'aluminium et de lithium. Le dérivé nitroso (VI) s'obtient par réaction d'un composé de formule :

HNR₅R₆ (VII)

dans laquelle R₅ et R₆ sont tels que définis ci-dessus pour (I), avec le nitrite de sodium en solution aqueuse en présence d'un acide tel que l'acide acétique.

Les composés de formule (VII) sont connus ou préparés par des méthodes connues.

Plus particulièrement on peut obtenir les amines HNR₁NR₅R₆ dans lesquelles R₁ = CH₃ selon le procédé décrit par Zinner et al., Arch. Pharm. (Weinheim, Ger.), 1966, 299, 245-248 et les amines HNR₁NR₅R₆ dans lesquelles R₁ = (C₂-C₆)alkyle selon le procédé décrit dans le brevet DE 2 409 431 à partir des amines HNR₁NR₅R₆ dans lesquelles R₁ = H.

Le 2-amino-2-azabicyclo[2.2.2]oct-5-ène se prépare selon Chem. Ber., 1986, 119, 1413-1423.

Le chlorure de 2-amino-2-méthyl-2-azoniabicyclo[2.2.2]oct-5-ène se prépare selon Chem. Ber., 1989, 122, 1153.

Le 2-amino-2-azaadamantane se prépare à partir du 2-azaadamantane, via le dérivé nitroso. Le 2-azaadamantane se prépare selon J. Org. Chem., 1981, 46, 4953.

Le mésitylènesulfonate du 1-amino-1-azoniabicyclo[2.2.2]octane se prépare selon J. Heterocyclic Chem., 1980, 17, 1241.

Les composés de formule (I) possèdent une très bonne affinité *in vitro* pour les récepteurs aux cannabinoïdes centraux et/ou périphériques, dans les conditions expérimentales décrites par Devane et al., Molecular Pharmacology, 1988, 34, 605-613.

Les composés selon l'invention possèdent également une affinité pour les récepteurs aux cannabinoïdes présents sur des préparations d'organes isolés stimulés électriquement. Ces essais ont été réalisés sur l'iléon de cobaye et sur le vas deferens de souris selon Roselt et al., Acta Physiologica, Scandinavia, 1975, 94, 142-144 et selon Nicolau et al., Arch. Int. Pharmacodyn., 1978, 236, 131-136.

La toxicité des produits selon l'invention est compatible avec leur utilisation en tant que médicament. Ainsi ils peuvent être utilisés pour leur activité psychotrope, notamment pour le traitement des troubles thymiques, des troubles anxieux, des troubles de l'humeur, du vomissement, des troubles mnésiques, des troubles cognitifs, des neuropathies, de la migraine, du stress, des maladies d'origine psychosomatique, de l'épilepsie, des dyskinésies ou de la maladie de Parkinson.

Les composés selon l'invention peuvent être également être utilisés en tant que médicament pour le traitement des troubles de l'appétit notamment en tant qu'anorexigène, pour le traitement de la schizophrénie, des troubles délirants, des troubles psychotiques en général, ainsi que des troubles liés à l'utilisation des substances psychotiques.

De plus selon l'invention les composés possèdent une utilité en tant qu'immunomodulateurs, dans la myorelaxation, l'asthme, l'épilepsie, le glaucome, ainsi que dans la chimiothérapie anticancéreuse, dans l'ischémie et l'angor, dans l'hypotension orthostatique et dans l'insuffisance cardiaque.

Les composés selon l'invention sont généralement administrés en unité de dosage.

Lesdites unités de dosage sont de préférence formulées dans des compositions pharmaceutiques dans lesquelles le principe actif est mélangé avec au moins un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables ou un de leurs solvates.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Dans les compositions pharmaceutiques de la présente invention, le principe actif est généralement formulé en unités de dosage contenant de 0,5 à 1000 mg, avantageusement de 1 à 500 mg, de préférence de 2 à 200 mg dudit principe actif par unité de dosage pour les administrations quotidiennes.

Lorsque l'on prépare une composition solide sous forme de comprimé, on mélange le principe actif principal avec un véhicule pharmaceutique tel que la silice, l'amidon, le lactose, le stéarate de magnésium, le talc ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le polyéthylèneglycol.

Le principe actif peut être formulé également sous forme de microcapsules ou microsphères éventuellement avec un ou plusieurs supports ou additifs.

Le principe actif peut être également présenté sous forme de complexe avec une cyclodextrine, par exemple α-, β- ou γ-cyclodextrine, 2-hydroxypropyl-β-cyclodextrine ou méthyl-β-cyclodextrine.

Les exemples suivants illustrent l'invention sans toutefois la limiter.

Les points de fusion ou de décomposition des produits, F, ont été mesurés en tube capillaire avec un appareil de Tottoli.

Dans les préparations et dans les exemples, les abréviations suivantes sont utilisées :
THF : tétrahydrofurane
Ether : éther diéthylique
Ether iso : éther diisopropylique
EtOH : éthanol
AcOEt : acétate d'éthyle
Et : éthyle
nPr : n-propyle
Ph : phényle
Me : méthyle
MeOH : méthanol
DCM: dichlorométhane
KOH : potasse
AcOH : acide acétique
HCl : acide chlorhydrique
NaCl : chlorure de sodium
TA : température ambiante
F : point de fusion.

### Préparations

### Préparation 1.1

### Acide 5-(4-chlorophényl)-1-(2,4-dichlorophényl)-4-méthylpyrazole-3-carboxylique.

A) Sel de lithium du 4-(4-chlorophényl)-3-méthyl-4-oxydo-2-oxobut-3-énoate d'éthyle.
   On ajoute, sous atmosphère d'azote, 125 ml d'une solution 1M du sel de lithium de l'hexaméthyldisilazane dans le THF, à 500 ml d'éther. On refroidit à-78°C et ajoute, goutte à goutte, une solution de 21 g de 4-chloropropiophénone dans 100 ml d'éther. Après 45 minutes d'agitation, on ajoute rapidement 19,2 ml d'oxalate d'éthyle et laisse 16 heures sous agitation en laissant remonter la température à TA. On filtre le précipité formé, lave à l'éther et sèche sous vide. On obtient 12,6 g du produit attendu.
B) Ester éthylique de l'acide 5-(4-chlorophényl)-1-(2,4-dichlorophényl)-4-méthylpyrazole-3-carboxylique.
   A une solution de 12,6 g du sel de lithium obtenu à l'étape précédente dans 70 ml d'EtOH on ajoute 9,8 g de 2,4-dichlorophénylhydrazine et laisse 16 heures sous agitation à TA. On filtre le précipité formé, lave à l'EtOH puis à l'éther et sèche sous vide. On obtient 12,6 g d'hydrazone que l'on dissout dans 100 ml d'AcOH. On chauffe à reflux pendant 24 heures puis verse le mélange réactionnel dans 500 ml d'eau glacée. On extrait à l'AcOEt, lave à l'eau, par une solution saturée de NaCl, sèche sur sulfate de magnésium et évapore sous vide. On obtient 9,6 g du produit attendu après cristallisation dans l'éther iso. F = 124°C.
C) Acide 5-(4-chlorophényl)-1-(2,4-dichlorophényl)-4-méthylpyrazole-3-carboxylique.
   A une solution de 9,6 g de l'ester obtenu à l'étape précédente dans 70 ml de MeOH on ajoute une solution de 3,3 g de KOH dans 70 ml d'eau et chauffe à reflux pendant 3 heures. On verse le mélange réactionnel sur 200 ml d'eau glacée, acidifie à pH = 1 par ajout d'une solution à 10 % d'HCl, filtre le précipité formé, lave à l'eau et sèche sous vide. On obtient 8,8 g de l'acide attendu. F = 211°C.

En procédant selon la préparation 1 ci-dessus, on prépare les esters et acides décrits dans le tableau 1 ci-dessous et utiles pour la synthèse des dérivés décrits dans le tableau 3.

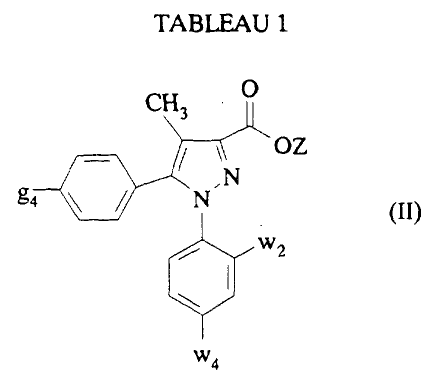

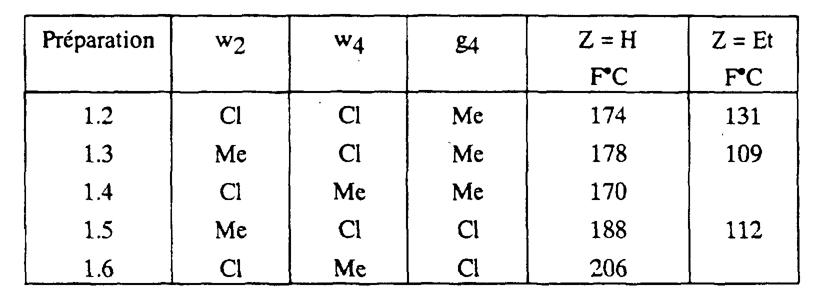

### Préparation 1.7

### Acide 5-(4-chlorophényl)-1-(2,4-dichlorophényl)-pyrazole-3-carboxylique.

A) Sel de sodium du 4-(4-chlorophényl)-4-oxydo-2-oxo-but-3-énoate de méthyle.
   12 g de sodium sont dissous dans 250 ml de méthanol anhydre. On ajoute ensuite un mélange de 64,6 ml de 4-chloroacétophénone et 67,1 ml d'oxalate de diéthyle dans 600 ml de méthanol en maintenant la température inférieure à 10°C. Le mélange réactionnel est ensuite agité à température ambiante pendant 3 heures puis on ajoute 1 l d'éther sec. On agite encore pendant 20 minutes, filtre, lave le précipité à l'éther et le sèche sous vide pour obtenir 74,6 g du sel de sodium attendu.
B) Ester méthylique de l'acide 5-(4-chlorophényl)-1-(2,4-dichlorophényl)-pyrazole-3-carboxylique.
   Une suspension de 26,3 g du sel de sodium obtenu précédemment et 23,5 g de chlorhydrate de 2,4-dichlorophénylhydrazine dans 250 ml d'acide acétique est chauffée à reflux pendant 4 heures. Après refroidissement, le mélange est versé sur 250 g de glace, les cristaux obtenus sont filtrés, lavés à l'eau et séchés sous vide pour fournir 26,3 g d'ester. F = 167°C.
C) Acide 5-(4-chlorophényl)-1-(2,4-dichlorophényl)pyrazole-3-carboxylique.
   Une solution de 3,70 g de KOH dans 35 ml d'eau est ajoutée à une solution de 10,0 g d'ester obtenu précédemment dans 35 ml de méthanol. Le mélange est chauffé à reflux pendant 4 heures, refroidi à température ambiante et versé dans 100 ml d'eau puis neutralisé par une solution d'HCl à 5 %. Les cristaux obtenus sont filtrés, lavés à l'eau puis au pentane et séchés sous vide. On obtient 9,50 g d'acide. F = 185°C.

En procédant selon la préparation 1.7 ci-dessus on prépare les acides décrits dans le tableau 2 ci-dessous.

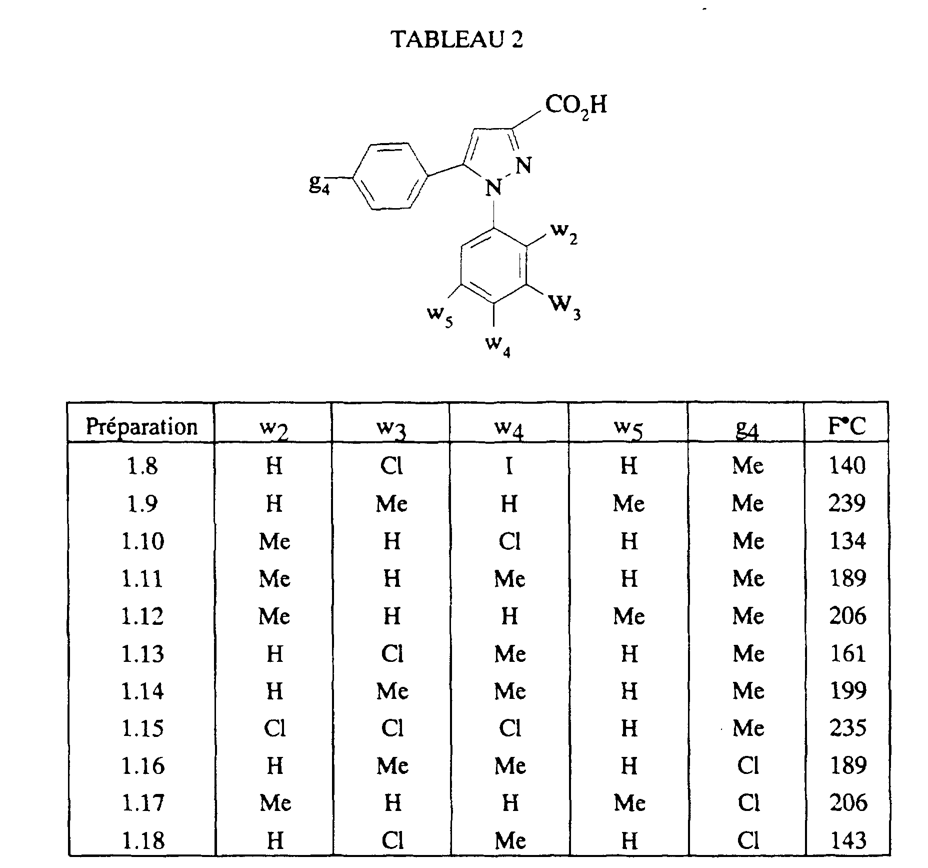

### Préparation 1.19

### Acide 5-(4-chlorophényl)-1-(2,4-dichlorophényl)-4-éthylpyrazole-3-carboxylique.

A) Ester éthylique de l'acide 5-(4-chlorophényl)-1-(2,4-dichlorophényl)-4-bromométhylpyrazole-3-carboxylique.
   A une solution de 1,62 g de l'ester éthylique de l'acide 5-(4-chlorophényl)-1-(2,4-dichlorophényl)-4-méthylpyrazole-3-carboxylique décrit dans la préparation 1.1, étape B dans 20 ml de CCl₄ on ajoute 0,70 g de N-bromosuccinimide et une pointe de spatule de péroxyde de benzoyle. Le mélange est chauffé à reflux pendant 16 heures puis le précipité blanc est filtré, le filtrat est lavé à l'eau puis par une solution saturée de NaCl. On obtient 2,20 g de mousse jaune que l'on cristallise dans l'éther iso pour obtenir 1,00 g de cristaux blancs, F = 108°C.
B) Ester éthylique de l'acide 5-(4-chlorophényl)-1-(2,4-dichlorophényl)-4-éthylpyrazole-3-carboxylique.
   A une suspension de 0,34 g de bromure cuivreux dans 20 ml d'éther éthylique refroidie à -20°C sous azote, on ajoute goutte à goutte 2,97 ml d'une solution de 1,6 M de méthyllithium dans l'éther. On agite encore 15 minutes à-20°C puis on refroidit à -40°C et on ajoute goutte à goutte une solution de 1,00 g de l'ester obtenu dans l'étape précédente dans 20 ml d'éther. On laisse lentement revenir à TA et agite encore 3 heures avant d'hydrolyser par 50 ml de solution saturée de NH₄Cl. Le mélange est extrait à l'éther et lavé à l'eau puis par une solution saturée de NaCl. On obtient 0,82 g de solide vert pâle que l'on chromatographie sur colonne de silice en éluant par le mélange AcOEt/hexane (15/85 ; v/v). On obtient 0,35 g de cristaux blancs, F = 105°C.
C) Acide 5-(4-chlorophényl)-1-(2,4-dichlorophényl)-4-éthylpyrazole-3-carboxylique.
   Une solution de 0,78 g de potasse dans 20 ml d'eau est ajoutée à une solution de 2,35 g d'ester obtenu à l'étape précédente dans 20 ml de méthanol et le mélange est chauffé à reflux pendant 3 heures. Le mélange est coulé sur 100 ml d'eau glacée puis acidifié à pH = 1 par addition d'HCl à 10 %. Le solide blanc obtenu est filtré, lavé à l'eau et séché sous vide. On obtient 2,58 g de l'acide attendu, F = 215°C.

### Préparation 1.20

### Acide 5-(4-chlorophényl)-1-(2,4-dichlorophényl)-4-pentylpyrazole-3-carboxylique.

A une suspension de 2,04 g d'iodure cuivreux dans 20 ml d'éther éthylique refroidie à -40°C sous azote, on ajoute goutte à goutte 13,2 ml d'une solution 1,6M de butyllithium dans l'hexane. On refroidit à -70°C et ajoute goutte à goutte une solution de 1,50 g d'ester méthylique de l'acide 5-(4-chlorophényl)-1-(2,4-dichlorophényl)-4-bromométhylpyrazole-3-carboxylique obtenu selon la Préparation 1.19 dans 20 ml d'éther. On laisse lentement revenir à TA et agite encore 3 heures avant d'hydrolyser par 50 ml de solution saturée de NH₄Cl. Le mélange est extrait à l'éther et lavé à l'eau puis par une solution saturée de NaCl. Après séchage sur MgSO₄ et évaporation des solvants on obtient 1,37 g d'huile visqueuse que l'on chromatographie sur colonne de silice en éluant par le mélange AcOEt/hexane (10/90; v/v). On obtient 0,42 g d'huile visqueuse (mélange d'esters méthyliques et butyliques). Une solution de 0,24 g de potasse dans 15 ml d'eau est ajoutée à une solution de 0,42 g du produit précédent dans 15 ml de méthanol et le mélange est chauffé à reflux pendant 3 heures. Le mélange est coulé sur 100 ml d'eau glacée puis acidifié à pH = 1 par addition d'HCl à 10 %. Le solide blanc est filtré, lavé à l'eau et séché sous vide. On obtient 0,39 g de l'acide attendu, F = 187°C.

### Préparation 2.1.

### Chlorhydrate de 1-amino-1,2,3,6-tétrahydropyridine.

A) 1-Nitroso-1,2,3,6-tétrahydropyridine.
   On refroidit à 0°C une solution de 2,0 ml de 1,2,3,6-tétrahydropyridine dans 6 ml d'eau et ajoute 16 ml d'AcOH puis, goutte à goutte, une solution de 9,1 g de nitrite de sodium dans 20 ml d'eau. On laisse 16 heures sous agitation à TA puis extrait au DCM, lave par une solution saturée d'hydrogénocarbonate de sodium, par une solution saturée de chlorure de sodium, sèche sur sulfate de magnésium et évapore sous vide le solvant. On obtient 2,34 g d'huile jaune que l'on utilise telle quelle à l'étape suivante.
B) Chlorhydrate de 1-amino-1,2,3,6-tétrahydropyridine.
   On refroidit à 0°C une solution de 2,34 g du composé obtenu à l'étape précédente dans 80 ml d'éther et ajoute, goutte à goutte, une suspension de 2,38 g d'hydrure de lithium et d'aluminium dans 60 ml d'éther. On chauffe à reflux pendant 3 heures puis refroidit le mélange réactionnel à 0°C et ajoute 2,3 ml d'eau puis 2,3 ml d'une solution aqueuse à 15 % de NaOH et enfin 6,9 ml d'eau. On filtre le précipité formé, lave ce dernier à l'éther puis reprend le filtrat à l'éther, lave par une solution saturée de chlorure de sodium, sèche sur sulfate de magnésium et évapore sous vide le solvant. On dissout l'huile obtenue dans 15 ml d'éther et ajoute goutte à goutte une solution saturée d'HCl gazeux dissout dans l'éther jusqu'à pH = 1. On filtre le précipité blanc formé, lave à l'éther et sèche sous vide. On obtient 1,84 g du chlorhydrate attendu. F = 142°C.
   Il est à noter que l'étape de réduction par LiAlH₄ peut présenter un temps d'amorçage assez long puis démarrer rapidement de façon très exothermique.

### Préparation 2.2 à 2.10

La 1-amino-4-phénylpipéridine (F = 65°C), le chlorhydrate de 1-amino-3-azabicyclo(3.2.2)nonane (F = 210°C), les cis et trans 1-amino-3,5-diméthylpipéridines (liquide), la 1-amino-3,3-diméthylpipéridine (liquide), la 1-amino-4-méthoxypipéridine (F = 147°C), le chlorhydrate de 1-amino-8-azabicyclo(4.3.0)non-3-ène (F = 84°C), le chlorhydrate de 1-amino-8-azaspiro(4.5)décane (F = 102°C), le 2-amino-2-azaadamantane (F = 90° C), sont préparés au départ des amines correspondantes selon le mode opératoire décrit aux étapes A et B de la Préparation 2.1. goutte à goutte une solution saturée d'HCl dans l'éther jusqu'à pH acide, puis décante l'huile obtenue et la sèche sous vide. On obtient 2,10 g de solide blanc, F = 107°C.

### EXEMPLE 1

### Chlorhydrate de N-(1,2,3,6-tétrahydropyridin-1-yl)-5-(4-chlorophényl)-1-(2,4-dichlorophényl)-4-méthylpyrazole-3-carboxamide.

A) Chlorure de l'acide 5-(4-chlorophényl)-1-(2,4-dichlorophényl)-4-méthylpyrazole-3-carboxylique.
   A une suspension de 8,8 g de l'acide obtenu à la Préparation 1.1 dans 90 ml de toluène on ajoute 5 ml de chlorure de thionyle et chauffe à reflux pendant 3 heures. On évapore sous vide à siccité le mélange réactionnel, reprend le résidu dans 90 ml de toluène et évapore de nouveau sous vide. On obtient 8 g de chlorure d'acide attendu qui est utilisé tel quel à l'étape suivante.
B) Chlorhydrate de N-(1,2,3,6-tétrahydropyridin-1-yl)-5-(4-chlorophényl)-1-(2,4-dichlorophényl)-4-méthylpyrazole-3-carboxamide.
   On refroidit à 0°C une solution de 0,59 g de chlorhydrate de 1-amino-1,2,3,6-tétrahydropyridine (obtenu à la préparation 2.1), 1,45 ml de triéthylamine dans 20 ml de DCM et ajoute, goutte à goutte, une solution de 1,32 g du chlorure de l'acide 5-(4-chlorophényl)-1-(2,4-dichlorophényl)-4-méthylpyrazole-3-carboxylique obtenu à l'étape précédente dans 20 ml de DCM. On laisse 3 heures sous agitation en laissant remonter la température à TA puis verse sur 100 ml d'eau glacée. On extrait au DCM, lave à l'eau, puis par une solution saturée de NaCl, sèche sur sulfate de magnésium et évapore sous vide le solvant. On chromatographie sur silice en éluant par le mélange toluène/AcOEt (90/10 ; v/v). On obtient 1,0 g de mousse incolore que l'on dissout dans 15 ml d'éther et ajoute goutte à goutte, une solution saturée d'HCl gazeux dans l'éther jusqu'à pH = 1. On filtre le précipité blanc formé, le lave à l'éther et le sèche sous vide. On obtient 0,55 g du chlorhydrate attendu. F = 200°C (décomposition).

En procédant selon l'exemple 1 on prépare les produits décrits dans le tableau 3 ci-après :

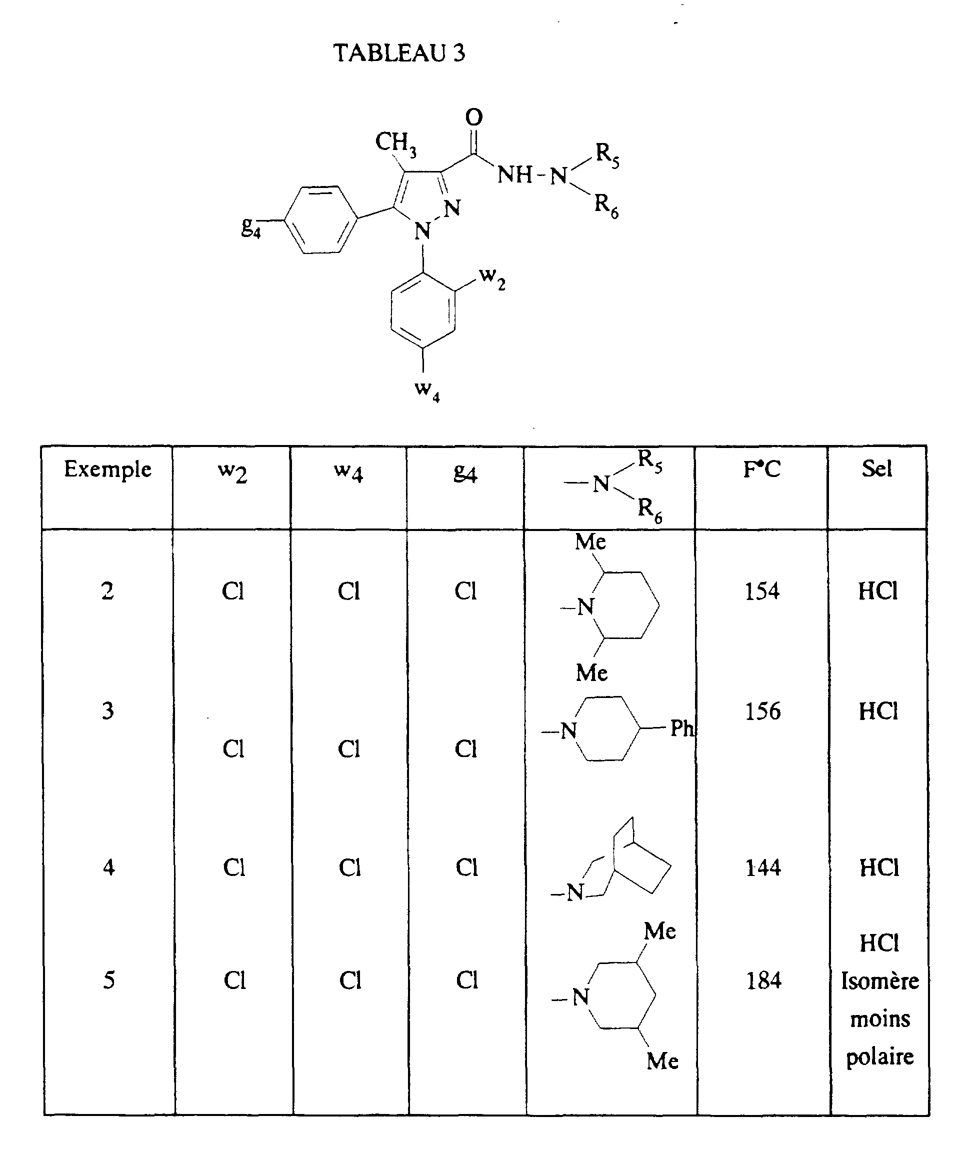

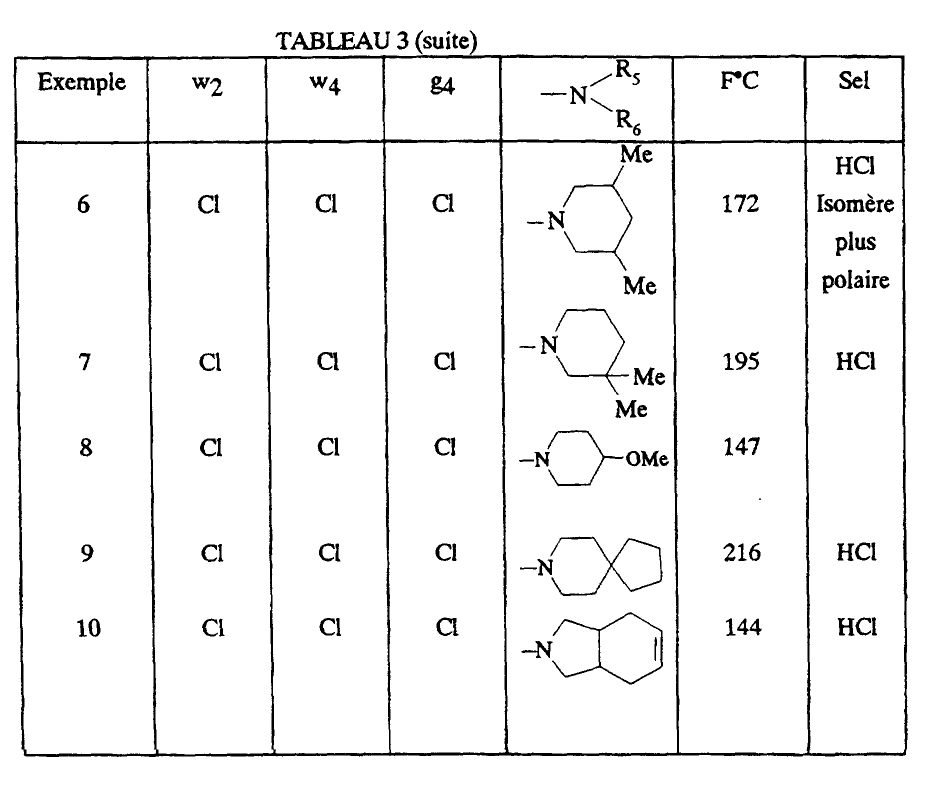

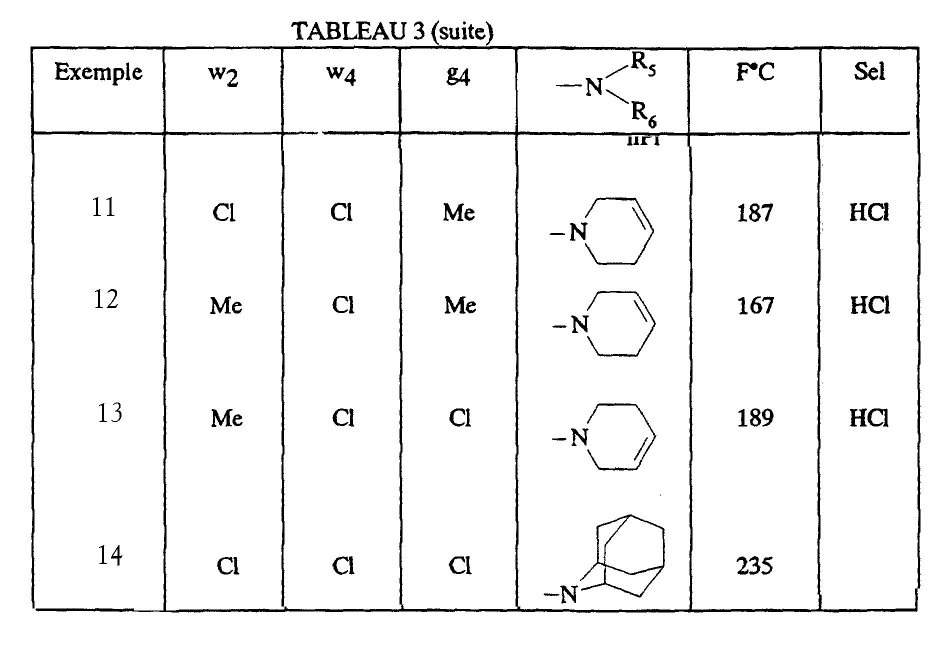

### EXEMPLE 15

### Chlorhydrate de N-(2,6-diméthylpipéridin-1-yl)-5-(4-chlorophényl)-1-(2,4-dichlorophényl)pyrazole-3-carboxamide.

A) Chlorure de l'acide 5-(4-chlorophényl)-1-(2,4-dichlorophényl)pyrazole-3-carboxylique.
   A une suspension de 9,50 g de l'acide obtenu à la Préparation 1.7 dans 100 ml de toluène on ajoute 5,8 ml de chlorure de thionyle et chauffe à reflux pendant 3 heures. On évapore sous vide à siccité le mélange réactionnel, reprend le résidu dans 50 ml de toluène et évapore sous vide le solvant. On obtient 8,28 g de chlorure d'acide attendu qui est utilisé tel quel à l'étape suivante.
B) Chlorhydrate de N-(2,6-diméthylpipéridin-1-yl)-5-(4-chlorophényl)-1-(2,4-dichlorophényl)pyrazole-3-carboxamide.
   On prépare ce composé selon le mode opératoire décrit à l'EXEMPLE 1 à partir de 1-amino-2,6-diméthylpipéridine et du chlorure de l'acide 5-(4-chlorophényl)-1-(2,4-dichlorophényl)pyrazole-3-carboxylique. F = 195°C.

### EXEMPLE 16

### Iodure de 1-méthyl-1-[5-(4-chlorophényl)-1-(2,4-dichlorophényl)-4-méthylpyrazole-3-carboxamido]pipéridinium et chlorure de 1-méthyl-1-[5-(4-chlorophényl)-1-(2,4-dichlorophényl)-4-méthylpyrazole-3-carboxamido]-pipéridinium.

A) N-(pipéridin-1-yl)-5-(4-dichlorophényl)-1-(2,4-dichlorophényl)-4-méthyl-pyrazole-3-carboxamide.
   On prépare ce composé selon le mode opératoire décrit à l'EXEMPLE 1 à partir de 1-aminopipéridine et du chlorure de l'acide 5-(4-chlorophényl)-1-(2,4-dichlorophényl)-4-méthylpyrazole-3-carboxylique. F = 148°C.
B) Iodure de 1-méthyl-1-[5-(4-chlorophényl)-1-(2,4-dichlorophényl)-4-méthylpyrazole-3-carboxamido]pipéridinium.
   A une solution de 0,23 g du composé obtenu à l'étape précédente dans 5 ml d'acétone on ajoute 0,35 g d'iodure de méthyle et chauffe à reflux pendant 24 heures. On évapore sous vide le solvant et reprend le résidu à l'éther. On filtre le précipité formé, le lave à l'éther et le sèche sous vide. On obtient 0,16 g de l'iodure attendu. F = 136°C (décomposition).
C) Chlorure de 1-méthyl-1-(5-(4-chlorophényl)-1-(2,4-dichlorophényl)-4-méthylpyrazole-3-carboxamido)pipéridinium.
   Une solution de 0,50 g de l'amide obtenu à l'étape A) dans 3,00 g d'iodure de méthyle est agitée à TA pendant 72 heures. Le solvant est évaporé sous vide, le résidu est repris dans un mélange MeOH-H₂O (10/90 ; v/v) et élué avec le même mélange sur une colonne de résine Amberlite IRA400.HCl. Le produit obtenu est cristallisé dans un mélange éther-éther iso. On obtient 0,26 g de solide blanc, F = 210°C.

### EXEMPLE 17

### Mésitylènesulfonate de N-(5-(4-chlorophényl)-1-(2,4-dichlorophényl)-4-méthylpyrazole-3-carboxamido)quinuclidinium.

3 ml d'une solution 1M d'éthylate de sodium dans l'éthanol sont évaporés à sec sous vide et le résidu blanc obtenu est repris dans 7 ml de THF anhydre. A cette solution on ajoute 1,00 g de mésitylènesulfonate de 1-aminoquinuclidinium (préparé selon J. Heterocycl. Chem. 1980, 17, 1241) et agite 2 heures à TA puis on ajoute 1,2 g de l'ester décrit dans la préparation 1.1, étape B et agite le mélange à TA pendant 5 jours. Le solide résiduel est filtré, les eaux-mères sont évaporées et le résidu est repris dans 15 ml de DCM. Cette solution est lavée par une solution d'HCl 0,1N puis une solution saturée de NaCl. Après séchage sur MgSO₄ et évaporation sous vide on obtient un solide blanc que l'on recristallise dans un mélange éther iso/acétone (90/10; v/v). On obtient 70 mg de solide cristallisé, F = 154°C.

### EXEMPLE 18

### Chlorhydrate de N-(1,2,3,6-tétrahydropyridin-1-yl)-5-(4-chlorophényl)-1-(2,4-dichlorophényl)-4-éthylpyrazole-3-carboxamide.

A) Chlorure de l'acide 5-(4-chlorophényl)-1-(2,4-dichlorophényl)-4-éthylpyrazole-3-carboxylique.
   A une suspension de 2,58 g de l'acide obtenu à la préparation 1.19 dans 20 ml de toluène on ajoute 1,43 ml de chlorure de thionyle, et le mélange est chauffé à reflux pendant 3 heures. Le mélange réactionnel est évaporé à sec sous vide et le résidu est repris dans 20 ml de toluène que l'on évapore sous vide (deux fois de suite). On obtient 2,28 g du chlorure d'acide attendu.
B) Chlorhydrate de N-(1,2,3,6-tétrahydropyridin-1-yl)-5-(4-chlorophényl)-1-(2,4-dichlorophényl)-4-éthylpyrazole-3-carboxamide.
   Une solution de 0,61 g du chlorure d'acide précédent dans 10 ml de DCM est ajoutée goutte à goutte à une solution de 0,21 g de chlorhydrate de 1-amino-1,2,3,6-tétrahydropyridine et 0,43 ml de triéthylamine dans 10 ml de DCM refroidie à 0°C. Le mélange réactionnel est agité à TA pendant 3 heures puis il est versé dans 100 ml d'eau glacée. La phase organique est extraite au DCM et lavée à l'eau puis par une solution de NaCI saturée et séchée sur MgSO₄. Par évaporation des solvants on obtient un produit brut que l'on purifie par cristallisation dans l'éther iso. On obtient 0,35 g du composé attendu. A une solution de 0,35 g de ce composé dans 10 ml d'éther on ajoute goutte à goutte une solution d'HCl gazeux dans l'éther jusqu'à pH acide. Le précipité blanc formé est filtré, lavé à l'éther et séché sous vide. On obtient 0,27 g du chlorhydrate attendu, F = 205°C (décomposition).

### EXEMPLE 19

### N-(1,2,3,6-tétrahydropyridin-1-yl)-5-(4-chlorophényl)-1-(2,4-dichlorophényl)-4-pentylpyrazole-3-carboxamide.

A) Chlorure de l'acide 5-(4-chlorophényl)-1-(2,4-dichlorophényl)-4-pentylpyrazole-3-carboxylique.
   A une suspension de 0,39 g de l'acide obtenu à la préparation 1.20 dans 8 ml de toluène on ajoute 0,50 ml de chlorure de thionyle, et le mélange est chauffé à reflux pendant 3 heures. Le mélange réactionnel est évaporé à sec sous vide et le résidu est repris dans 20 ml de toluène que l'on reévapore sous vide (deux fois de suite). On obtient ainsi 0,44 g du chlorure d'acide attendu.
B) N-(1,2,3,6-tétrahydropyridin-1-yl)-5-(4-chlorophényl)-1-(2,4-dichlorophényl)-4-pentylpyrazole-3-carboxamide.
   Une solution de 0,22 g du chlorure d'acide précédent dans 8 ml de DCM est ajoutée goutte à goutte à une solution de 0,061 g de 1-amino-1,2,3,6-tétrahydropyridine et 0,079 ml de triéthylamine dans 10 ml de DCM refroidie à 0°C. Le mélange réactionnel est agité à TA pendant 16 heures puis il est versé dans 100 ml d'eau glacée. La phase organique est extraite au DCM et lavée à l'eau puis par une solution saturée de NaCl et séchée sur MgSO₄. Par évaporation des solvants on obtient un produit brut que l'on purifie par chromatographie sur colonne de silice en éluant par le mélange AcOEt/toluène (8/92 ; v/v) puis cristallisation dans l'éther iso. On obtient 0,12 g du produit attendu, F = 150°C.

## Revendications

1. Composé de formule : 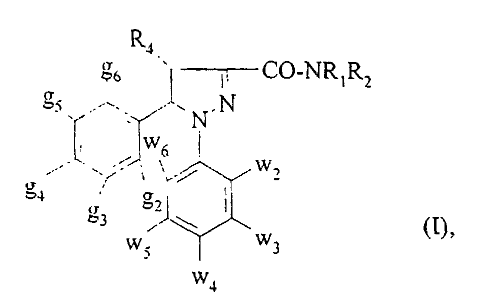 dans laquelle :
- g₂, g₃, g₄, g₅, g₆ et w₂, w₃, w₄, w₅, w₆ sont identiques ou différents, et représentent indépendamment l'hydrogène, un atome de chlore, de brome ou d'iode, un (C₁-C₃)alkyle, un (C₁-C₃)alcoxy, un trifluorométhyle, un groupe nitro et g₄ peut également représenter un groupe phényle ;
- R₁ représente un (C₁-C₆)alkyle ou un hydrogène ;
- R₂ représente -⁺NR₃R₅R₆ ou -NR₅R₆;
- R₃ représente un (C₁-C₆)alkyle ou R₃ forme un pont avec un des atomes du radical hétérocyclique formé par NR₅R₆ ;
- R₄ représente l'hydrogène ou un (C₁-C₅)alkyle ;
- R₅ et R₆ ensemble avec l'atome d'azote auquel ils sont liés constituent un radical hétérocyclique, saturé ou insaturé, de 5 à 10 chaînons, non substitué ou substitué une ou plusieurs fois par un alkyle en C₁-C₆, un benzyle, un phényle, un hydroxyle, un alcoxy en C₁-C₆, un halogène ;
à la condition que lorsque R₂ représente NR₅R₆, R₅ et R₆ ensemble avec l'atome d'azote auquel ils sont liés constituent un radical hétérocyclique autre qu'un radical saturé de 5 à 8 chaînons non substitué ou substitué par un (C₁-C₃)alkyle, un hydroxyle ou un benzyle ;
ses sels et leurs solvates.

2. Composé de formule (I) selon la revendication 1, dans lequel au moins un des substituants w₂, w₃, w₄, w₅, w₆, g₂, g₃, g₄, g₅ et g₆ est autre que l'hydrogène.

3. Composé de formule (I) selon la revendication 1 ou 2, dans lequel au moins un des substituants w₂, w₃, w₄, w₅, w₆, g₂, g₃, g₄, g₅ et g₆ est choisi parmi un chlore ou un méthyle.

4. Composé de formule (I) selon la revendication 1 ou 2, dans lequel w₂, w₄ et g₄ sont des atomes de chlore.

5. Composé de formule (I) selon l'une des revendications 1 à 4, dans lequel R₄ est H, méthyle ou éthyle.

6. Composé de formule (I) selon la revendication 1 ou 2, dans lequel w₂, w₄ et g₄ représentent chacun un chlore, R₁ représente l'hydrogène et R₄ représente un méthyle ou un éthyle.

7. Composé de formule (I) selon la revendication 1 ou 2, dans lequel g₄ est un chlore ou un méthyle, w₃ et w₄ ou w₂ et w₅ sont choisis parmi un chlore ou un méthyle, R₁ représente un méthyle, un éthyle ou un propyle et R₄ est l'hydrogène.

8. Composé de formule (I) selon l'une des revendications 1 à 7, dans lequel R₂ représente un radical hétérocyclique monoinsaturé à 5, 6 ou 7 chaînons.

9. Composé de formule (I) selon la revendication 8 dans lequel R₂ représente un groupe 1,2,3,6-tétrahydropyridin-1-yle.

10. Composé de formule : 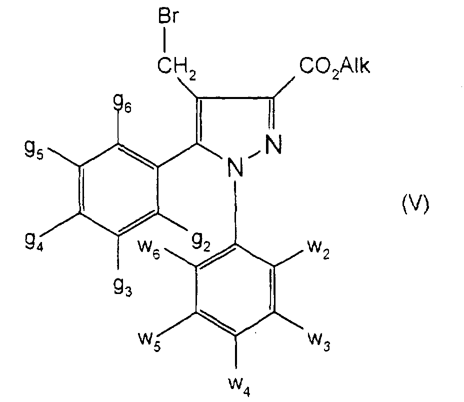 dans laquelle g₂, g₃, g₄, g₅, g₆, w₂, w₃, w₄, w₅ et w₆ sont tels que définis pour (I) dans la revendication 1, et Alk est un méthyle ou un éthyle.

11. Composé de formule (V) selon la revendication 10, qui est le 5-(4-chlorophényl)-1-(2,4-dichlorophényl)-4-bromométhylpyrazole-3-carboxylate d'éthyle.

12. Procédé pour la préparation d'un composé de formule (I) selon la revendication 1, de ses sels et de leurs solvates, caractérisé en ce qu'on traite un dérivé fonctionnel de l'acide pyrazole-3-carboxylique de formule : 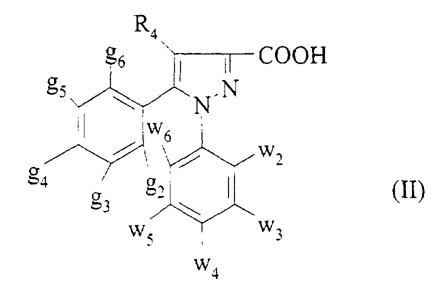 dans laquelle w₂, w₃, w₄, w₅, w₆, g₂, g₃, g₄, g₅, g₆ et R₄ sont tels que définis pour (I) dans la revendication 1 avec une amine de formule HNR₁R₂ dans laquelle R₁ et R₂ sont tels que définis pour (I) dans la revendication 1, et en ce qu'on transforme éventuellement le composé ainsi obtenu en l'un de ses sels ou de leurs solvates.

13. Composition pharmaceutique contenant, en tant que principe actif, un composé selon l'une des revendications 1 à 9, ou l'un de ses sels pharmaceutiquement acceptables.

14. Composition pharmaceutique selon la revendication 13, sous forme d'unité de dosage, dans laquelle le principe actif est mélangé à au moins un excipient pharmaceutique.

15. Composition pharmaceutique selon la revendication 14, contenant de 0,5 à 1000 mg de principe actif.

## Patentansprüche

1. Verbindung der Formel 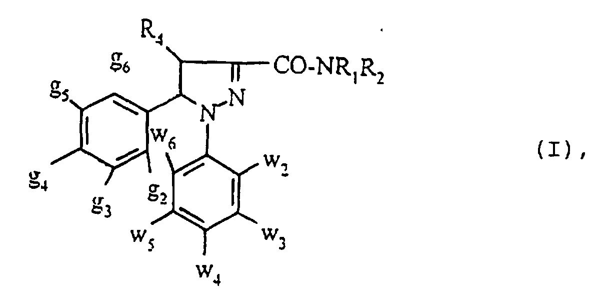 worin
- g₂, g₃, g₄, g₅, g₆ und w₂, w₃, w₄, w₅, w₆ gleich oder verschieden sind und unabhängig voneinander Wasserstoff, ein Chlor-, Brom- oder Iodatom, (C₁-C₃)-Alkyl, (C₁-C₃)-Alkoxy, Trifluormethyl oder eine Nitrogruppe darstellen und g₄ auch eine Phenylgruppe darstellen kann;
- R₁ (C₁-C₆)-Alkyl oder Wasserstoff darstellt;
- R₂ -⁺NR₃R₅R₆ oder -NR₅R₆ darstellt;
- R₃ (C₁-C₆)-Alkyl darstellt oder R₃ eine Brücke mit einem der Atome des von -NR₅R₆ gebildeten heterocyclischen Restes bildet;
- R₄ Wasserstoff oder (C₁-C₅)-Alkyl darstellt;
- R₅ und R₆ zusammen mit dem Stickstoffatom, an das sie ge-bunden sind, einen gesättigten oder ungesättigten 5- bis 10-gliedrigen heterocyclischen Rest bilden, der gegebenenfalls ein- oder mehrmals durch (C₁-C₆)-Alkyl, Benzyl, Phenyl, Hydroxyl, (C₁-C₆)-Alkoxy oder Halogen substituiert ist;
mit der Maßgabe, dass, wenn R₂ für NR₅R₆ steht, R₅ und R₆ zusammen mit dem Stickstoffatom, an das sie gebunden sind, einen heterocyclischen Rest darstellen, ausgenommen einen gesättigten 5- bis 8-gliedrigen Rest, der gegebenenfalls durch (C₁-C₃)-Alkyl, Hydroxyl oder Benzyl substituiert ist;
die Salze derselben und ihre Solvate.

2. Verbindung der Formel (I) nach Anspruch 1, worin mindestens einer der Substituenten w₂, w₃, w₄, w₅, w₆, g₂, g₃, g₄, g₅ und g₆ nicht Wasserstoff ist.

3. Verbindung der Formel (I) nach Anspruch 1 oder 2, worin mindestens einer der Substituenten w₂, w₃, w₄, w₅, w₆, g₂, g₃, g₄, g₅ und g₆ ausgewählt ist aus Chlor oder Methyl.

4. Verbindung der Formel (I) nach Anspruch 1 oder 2, worin w₂, w₄ und g₄ Chloratome sind.

5. Verbindung der Formel (I) nach einem der Ansprüche 1 bis 4, worin R₄ H, Methyl oder Ethyl ist.

6. Verbindung der Formel (I) nach Anspruch 1 oder 2, worin w₂, w₄ und g₄ jeweils Chlor darstellen, R₁ Wasserstoff darstellt und R₄ Methyl oder Ethyl darstellt.

7. Verbindung der Formel (I) nach Anspruch 1 oder 2, worin g₄ Chlor oder Methyl ist, w₃ und w₄ oder w₂ und w₅ ausgewählt sind aus Chlor oder Methyl, R₁ Methyl, Ethyl oder Propyl darstellt und R₄ Wasserstoff ist.

8. Verbindung der Formel (I) nach einem der Ansprüche 1 bis 7, worin R₂ einen mono-ungesättigten 5-, 6- oder 7-gliedrigen heterocyclischen Rest darstellt.

9. Verbindung der Formel (I) nach Anspruch 8, worin R₂ 1,2,3,6-Tetrahydropyridin-1-yl darstellt.

10. Verbindung der Formel: 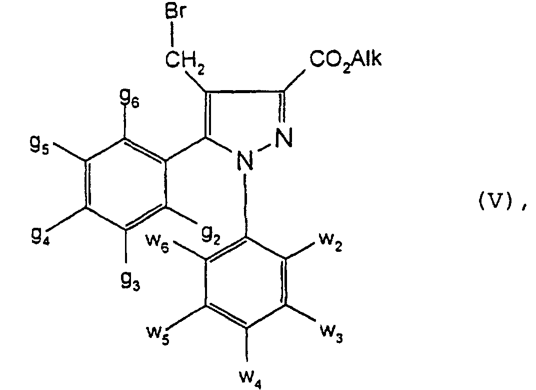 worin g₂, g₃, g₄, g₅, g₆, w₂, w₃, w₄, w₅ und w₆ wie in Anspruch 1 für (I) definiert sind und Alk für Methyl oder Ethyl steht.

11. Verbindung der Formel (V) nach Anspruch 10, nämlich 5-(4-Chlorphenyl )-1-(2,4-dichlorphenyl)-4-brommethylpyrazol-3-ethylcarboxylat.

12. Verfahren zur Herstellung einer Verbindung der Formel (I) nach Anspruch 1, der Salze derselben und ihrer Solvate, dadurch gekennzeichnet, dass ein funktionelles Derivat von Pyrazol-3-carbonsäure der Formel 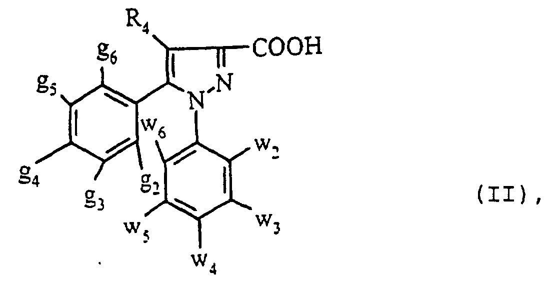 worin w₂, w₃, w₄, w₅, w₆, g₂, g₃, g₄, g₅, g₆ und R₄ wie in Anspruch 1 für (I) definiert sind, mit einem Amin der Formel HNR₁R₂, worin R₁ und R₂ wie in Anspruch 1 für (I) definiert sind, behandelt wird und die so erhaltene Verbindung gegebenenfalls in eines ihrer Salze oder deren Solvate übergeführt wird.

13. Pharmazeutische Zusammensetzung, die als Wirkstoff eine Verbindung nach einem der Ansprüche 1 bis 9 oder eines ihrer pharmazeutisch annehmbaren Salze enthält.

14. Pharmazeutische Zusammensetzung nach Anspruch 13 in Dosiseinheitsform, in welcher der Wirkstoff mit mindestens einem pharmazeutischen Exzipienten vermischt ist.

15. Pharmazeutische Zusammensetzung nach Anspruch 14, die 0,5 bis 1000 mg Wirkstoff enthält.

## Claims

1. A compound of the formula 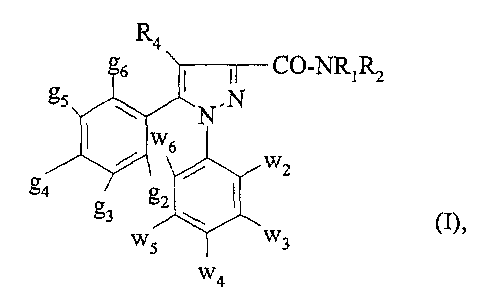 in which:
- g₂, g₃, g₄, g₅ and g₆, and w₂, w₃, w₄, w₅ and w₆, are identical or different and are independently hydrogen, a chlorine, bromine or iodine atom, a (C₁-C₃)alkyl, a (C₁-C₃)alkoxy, a trifluoromethyl or a nitro group and g₄ can also be a phenyl group;
- R₁ is a (C₁-C₆)alkyl or a hydrogen;
- R₂ is -⁺NR₃R₅R₆ or -NR₅R₆;
- R₃ is a (C₁-C₆)alkyl or R₃ forms a bridge with one of the atoms of the heterocyclic radical formed by NR₅R₆;
- R₄ is hydrogen or a (C₁-C₅)alkyl; and
- R₅ and R₆, together with the nitrogen atom to which they are bonded, form a 5- to 10-membered saturated or unsaturated heterocyclic radical which is unsubstituted or monosubstituted or polysubstituted by a (C₁-C₆)alkyl, a benzyl, a phenyl, a hydroxyl, a (C₁-C₆)alkoxy or a halogen,
with the proviso that when R₂ is NR₅R₆, R₅ and R₆, together with the nitrogen atom to which they are bonded, form a heterocyclic radical other than a 5- to 8- membered saturated radical which is unsubstituted or substituted by a (C₁-C₃)alkyl, a hydroxyl or a benzyl,
its salts and their solvates.

2. The compound according to claim 1 in which at least one of the substituents w₂, w₃, w₄, w₅, w₆, g₂, g₃, g₄, g₅ and g₆ is other than hydrogen.

3. The compound according to claim 1 or 2 in which at least one of the substituents w₂, w₃, w₄, w₅, w₆, g₂, g₃, g₄, g₅ and g₆ is selected from a chlorine and a methyl.

4. The compound according to claim 1 or 2 in which w₂, w₄ and g₄ are chlorine atoms.

5. The compound according to one of claims 1 to 4 in which R₄ is H, methyl or ethyl.

6. The compound of formula (I) according to claim 1 in which w₂, w₄ and g₄ are each a chlorine, R₁ is hydrogen, and R₄ is a methyl or an ethyl.

7. The compound of formula (I) according to claim 1 in which g₄ is a chlorine or a methyl, w₃ and w₄, or w₂ and w₅, are selected from chlorine and methyl, R₁ is a methyl, an ethyl or a propyl, and R₄ is hydrogen.

8. The compound according to one of claims 1 to 7 in which R₂ is a 5-, 6- or 7-membered monounsaturated heterocyclic radical.

9. The compound according to claim 8 in which R₂ is a 1,2,3,6-tetrahydropyridin-1-yl group.

10. A compound of the formula : 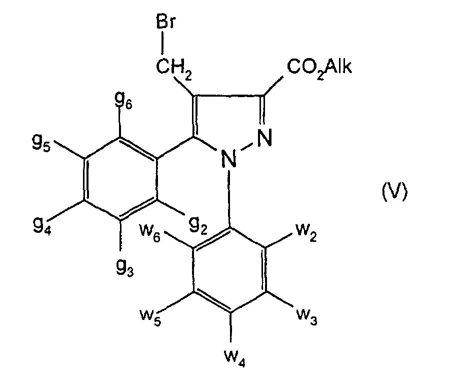 in which g₂, g₃, g₄, g₅, g₆, w₂, w₃, w₄, w₅ and w₆ are as defined for (I) in claim 1 and Alk is a methyl or an ethyl.

11. The compound of formula (V) according to claim 10, which is ethyl 5-(4-chlorophenyl)-1-(2,4-dichlorophenyl )-4-bromomethylpyrazole-3-carboxylate.

12. A process for the preparation of a compound of formula (I) according to claim 1, its salts and their solvates, which comprises treating a functional derivative of the pyrazole-3-carboxylic acid of the formula 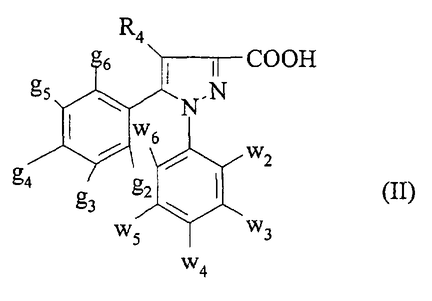 in which w₂, w₃, w₄, w₅, w₆, g₂, g₃, g₄, g₅, g₆ and R₄ are as defined for (I) in claim 1, with an amine of the formula HNR₁R₂, in which R₁ and R₂ are as defined for (I) in claim 1, and optionally converting the resulting compound to one of its salts or their solvates.

13. A pharmaceutical composition which contains a compound according to one of claims 1 to 9, or one of its pharmaceutically acceptable salts, as the active principle.

14. The pharmaceutical composition according to claim 13, which is in the form of a dosage unit, in which the active principle is mixed with at least one pharmaceutical excipient.

15. The pharmaceutical composition according to claim 14 which contains from 0.5 to 1000 mg of active principle.
